# EUROPEAN PATENT SPECIFICATION

(11) **EP 1 810 703 B1**
(45) Date of publication and mention of the grant of the patent: **07.10.2009**
(21) Application number: 06425024.4
(22) Date of filing: 20.01.2006
(51) Int. Cl.: A61M 1/16, A61M 1/34

(54) **Dialysis machine with arterial pressure monitoring by measuring blood oxygen saturation and sodium concentration**
Dialysegerät mit Blutdrucküberwachung durch Messung der Sauerstoffsättigung des Bluts und der Salzkonzentration
Machine de dialyse surveillant la pression artérielle par mesure de la saturation sanguine en oxygène et la concentration sodique

(43) Date of publication of application: 25.07.2007
(73) Proprietor: Bellco S.r.l., Mirandola (IT)
(72) Inventor: Baroni, Paolo, 46035 Ostiglia (IT); Cavani, Silvia, 41100 Modena (IT); Fiorenzi, Andrea, 41013 Castelfranco Emilia (IT)
(74) Representative: Cerbaro, Elena

(56) References cited:
- US-A- 4 370 983
- US-A- 4 844 871
- US-A- 5 230 341
- US-A- 5 499 627
- US-A1- 2002 068 015

## Description

The present invention relates to a dialysis machine with arterial pressure monitoring by measuring oxygen saturation.

Dialysis is a blood cleansing method capable of restoring the fluid-electrolyte balance and of eliminating water in excess and toxic substances which accumulate in the organism in course of kidney failure, rendering them to a liquid with an electrolytic content similar to that of normal plasma which does not contain them; here and below such liquid will be indicated with the term "dialysing solution". The application of such method envisages that, after being aspirated from the patient's arm, the blood flows along the so-called arterial line, and is let into the dialyser, at the output of which it flows along that known as venous line and returns, cleansed, to the patient.

The haemodiafiltration technique, to which the following description refers without for this loosing in generality, envisages that blood cleansing is achieved by exploiting both the phenomenon of diffusion and the phenomenon of convection. Cleansing by diffusion is due to a balancing of the blood and of the dialysing solution concentrations flowing on different sides of a semi permeable membrane. While, cleansing by convection occurs when a pressure gradient is obtained between the dialysing solution compartment and the blood compartment in favour of the latter. In this way, there is a passage of plasmatic water through the semi permeable membrane which by dragging determines also the passage of the toxic substances dissolved therein.

The dialysing solution does not contain the substances to be eliminated from the blood, such as urea, uric acid, creatine, phosphorous, etc., while it contains a precise amount of other substances to be rebalanced, such as sodium, calcium, magnesium, potassium, etc.

The haemodiafiltration treatment entails a series of potential risks which may cause cardiovascular system disorder due to deficiencies of the normal arterial pressure regulation mechanisms in the patient undergoing the treatment. Specifically, such deficiencies may lead the patient to cardiovascular failure.

It is the object of the present invention to achieve a dialysis machine, whose technical features are such to immediately detect possible cardiovascular response anomalies in the patient undergoing dialysis and to consequently and automatically intervene to prevent the failure.

US2002068015 disclose an apparatus and a method for alleviation of symptoms of inappropriate blood pressure and cramping from the removal of water during ultrafiltration hemodyalisis and hemofiltration treatment. The invention disclosed uses oxygen concentration measured by sensors in various points in the blood treatment circuitry to estimate parameters necessary to control the rate at which water is removed from the patient's blood.

US5230341 discloses a blood purification apparatus comprising an ultrasonic sensor which is configured in such a manner that at the and during the filtration the change of the ultrasonic signals is determined and the ultrafiltration rate and/or the mixture of the dialysing solution is changed accordingly.

The object of the present invention is a dialysis machine , the essential characteristics of which are specified in Claim 1, and preferred and/or auxiliary characteristics of which are specified in Claims 2-4.

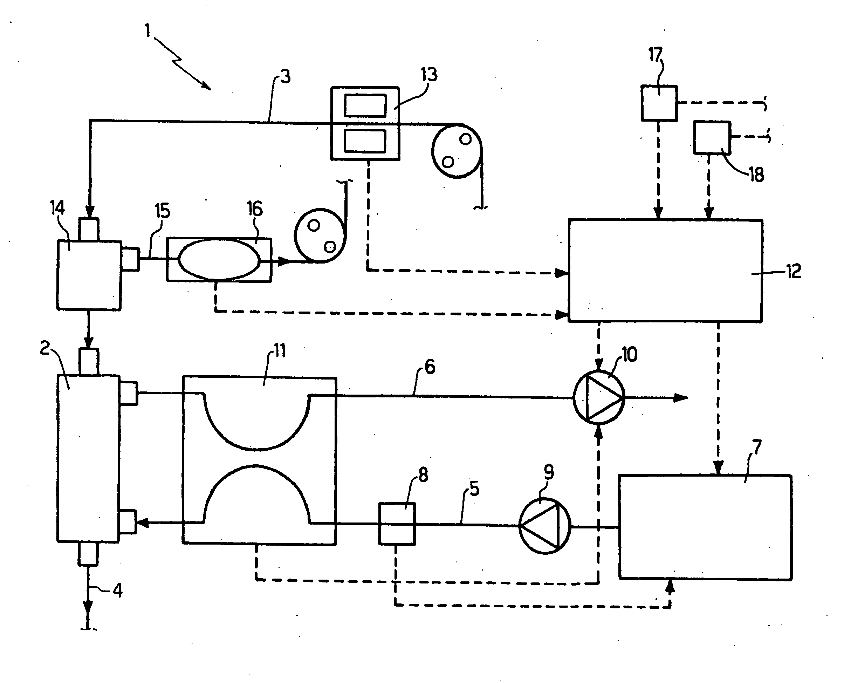

The following example is by way of non-limiting illustration for a better understanding of the invention with the help of the figure in the accompanying drawing, which is a schematic view of part of a preferred embodiment of the dialysis machine according to the present invention.

In the figure, it is indicated as a whole by 1 a dialysis machine (only partially shown) whose components are shown in schematic form.

The machine 1 comprises a haemodiafiltration filter 2 (known and not shown in detail), an arterial line 3 which is responsible for conveying the blood from the patient to the filter 2, a venous line 4 which is responsible for conveying the blood from the filter 2 to the patient, an inlet branch 5 and an outlet branch 6 of the dialysing solution respectively to and from the filter 2, a dialysing solution preparing device 7 connected to the input branch 5, a conductivity measurer 8 applied to the input branch 5 and connected to the preparing device 7, a pair of pumps 9 and 10 respectively applied to the input branch 5 and the output branch 6 of the dialysing solution, a differential flowmeter 11 described in patent EP1342479 by the Applicant, and a command/control unit 12 connected to the preparing device 7, the pump 10 and the differential flowmeter 11.

The machine 1 comprises an oxygen saturation measuring device 13 applied to the arterial line 3. In particular, the device 13 is capable of reading with optical means the oxyhaemoglobin fraction in the total haemoglobin, and consequently of providing an indication concerning the patient's cardiovascular response. The particular device 13 experimented by the Applicant is marketed by DATAMED S.r.L. under the trade name BLOP4.

The presence of the device 13 described above allows to continuously read the oxygen saturation in the patient's blood and to transmit the respective results to the unit 12 to which it is connected. The unit 12, in which haemodiafiltration process adaptation algorithms are implemented according to the oxygen saturation in the patient's blood, intervenes in particular on the ultrafiltering component of the filter 2, on the sodium concentration in the dialysing solution and on the temperature of the dialysing solution itself.

The machine 1 comprises an isolated ultrafiltering device 14 arranged along the arterial line 3, so as to produce an amount of ultrafiltrated plasma which is let into an arterial branch line 15. Generally, the arterial branch line 15 comprises a purification device by adsorption and reconnects to the arterial line 3 in the segment comprised between the isolated ultrafiltering device 14 and the haemodialysis filter 2. The machine 1 further comprises a blood sodium concentration measurer 16 applied to the arterial branch line 15 and connected to the command/control unit 12. The unit 12, in which algorithms for adapting the conductivity and sodium concentration values to be obtained in the dialysing solution according to the level of sodium in the patient's blood are implemented, intervenes on the dialysing solution through the preparing device 7 and the pump 10 for restoring the correct sodium value in the patient's blood, if necessary.

Finally, the machine 1 comprises a heart rate measuring device 17 and an arterial pressure measuring device 18, both connected to the command/control unit 12 to provide further information which will be processed by the unit according to particular programmed algorithms.

Obviously, the dialysis machine according to the present invention avoids that during dialysis treatment the patient can be subject to clinical complications due to deficiencies of the arterial pressure regulating mechanisms.

Furthermore, thanks to the presence of the sodium concentration measurer and auxiliary of the arterial and heart rate measuring devices, the patient is monitored even more completely during the treatment.

## Claims

1. A dialysis machine (1) comprising a haemodialysis filter (2), an input branch (5) of the dialysing solution into said filter (2), an output branch (6) of the dialysing solution from said filter (2), an arterial line (3) which is responsible for conveying the blood from the patient to the filter (2), a venous line (4) which is responsible for conveying the blood from the filter (2) to the patient, a plurality of pumps (9, 10) adapted to circulate both the blood and the dialysing solution, an oxygen saturation measuring device (13) applied to said arterial line (3) and a command/control unit (12) adapted to receive information from said oxygen saturation measuring device (13); said machine being **characterised in that** it comprises an isolated ultrafiltering device (14) arranged along the patient's arterial line (3), and a patient's blood sodium concentration measurer (16) that is applied to the ultrafiltrated plasma produced by said isolated ultrafiltering device (14) and adapted to give information to said command/control unit (12); said command/control unit (12) being connected to a dialysing solution preparing device (7) adapted to modify the composition and temperature of the dialysing solution itself, and to a pump (10) applied to said output branch (6) of the dialysing solution adapted to adjust the flow of ultrafiltrated taken from the patient; said command/control unit (12) having implemented haemodiafiltration process adaptation algorithms according to the oxygen saturation in the patient's blood and intervening on an ultrafiltering component of said filter (2), on the sodium concentration, and on the temperature of the dialysing solution; said command/control unit (12) further having implemented algorithms for adapting the conductivity and sodium concentration in the dialysing solution according to the level of sodium in the patient's blood and intervening on the dialysing solution through the dialysing solution preparing device (7) and on said pump (10) applied to said output branch for restoring the correct sodium value in the patient's blood.

2. A machine according to claim 1, **characterised in that** said blood oxygen saturation measuring device (13) comprises optical means for detecting the oxyhaemoglobin in the total haemoglobin.

3. A machine according to claim 1 or 2, **characterised in that** it comprises a device (18) for measuring the patient's arterial pressure; said device (18) for measuring the patient's arterial pressure being connected to said command/control unit (12).

4. A machine according to any of the preceding claims,
**characterised in that** it comprises a patient's heart rate measuring device (17); said patient's heart frequency measuring device (17) being connected to said command/control unit (12).

## Patentansprüche

1. Dialysemaschine (1), umfassend einen Hämodialysefilter (2), eine Eingangsverzweigung (5) der Dialyselösung in den Filter (2), eine Ausgangsverzweigung (6) der Dialyselösung aus dem Filter (2), eine arterielle Leitung (3), die verantwortlich ist für die Leitung des Bluts vom Patienten zum Filter (2), eine venöse Leitung (4), die verantwortlich ist für die Leitung des Bluts vom Filter (2) zum Patienten, eine Vielzahl an Pumpern (9, 10), die für eine Zirkulation von sowohl dem Blut als auch der Dialyselösung geeignet sind, eine Sauerstoffsättigungsmessvorrichtung (13), die an der arteriellen Leitung (3) angeordnet ist, und eine Befehls-/Steuereinheit (12), die zum Empfang von Informationen von der Sauerstoffsättigungsmessvorrichtung (13) geeignet ist, wobei die Maschine **dadurch gekennzeichnet ist, dass** sie eine isolierte Ultrafiltrationsvorrichtung (14), die entlang der arteriellen Leitung (3) des Patienten angeordnet ist, und einen Patientenblutnatriumkonzentrationsmessapparat (16), der auf das von der isolierten Ultrafiltrationsvorrichtung (14) erzeugte ultrafiltrierte Plasma angewendet wird und geeignet ist, der Befehls-/Steuereinheit (12) Informationen zu geben, umfasst, wobei die Befehls-/Steuereinheit (12) verbunden ist mit einer Dialyselösungsherstellungsvorrichtung (7), die zur Änderung der Zusammensetzung und Temperatur der Dialyselösung selbst geeignet ist, und mit einer Pumpe (10), die auf die Ausgangsverzweigung (6) der Dialyselösung angewendet wird und zur Einstellung des Durchflusses von vom Patienten genommenem Ultrafilriertem geeignet ist, wobei die Befehls-/Steuereinheit (12) einen implementierten Hämodiafiltrationsprozessadaptionsalgorithmus entsprechend der Sauerstoffsättigung im Blut des Patienten aufweist und bei einer Ultrafiltrationskomponente des Filters (2), bei der Natriumkonzentration und bei der Temperatur der Dialyselösung eingreift, wobei die Befehls-/Steuereinheit (12) ferner Algorithmen implementiert hat zur Anpassung der Leitfähigkeit und Natriumkonzentration in der Dialyselösung entsprechend dem Niveau an Natrium im Blut des Patienten und zum Eingreifen bei der Dialyselösung durch die Dialyselösungsherstellungsvorrichtung (7) und bei der auf die Ausgangsverzweigung angewendeten Pumpe (10), um den korrekten Natriumwert im Blut des Patienten wiederherzusteilen.

2. Maschine nach Anspruch 1, **dadurch gekennzeichnet, dass** die Sauerstoffsättigungsmessvorrichtung (13) optische Mittel zur Erfassung des Oxyhämoglobins im Gesamthämoglobin umfasst.

3. Maschine nach Anspruch 1 oder 2, **dadurch gekennzeichnet, dass** sie eine Vorrichtung (18) zur Messung des arteriellen Drucks des Patienten umfasst, wobei die Vorrichtung (18) zur Messung des arteriellen Drucks des Patienten mit der Befehls-/Steuereinheit (12) verbunden ist.

4. Maschine nach einem der vorhergehenden Ansprüche, **dadurch gekennzeichnet, dass** sie eine Vorrichtung (17) zur Messung der Herzfrequenz des Patienten umfasst, wobei die Vorrichtung (17) zur Messung der Herzfrequenz des Patienten mit der Befehls-/Steuereinheit (12) verbunden ist.

## Revendications

1. Machine de dialyse (1) comprenant un filtre d'hémodialyse (2), une ramification d'entrée (5) de la solution de dialyse dans ledit filtre (2), une ramification de sortie (6) de la solution de dialyse depuis ledit filtre (2), une branche artérielle (3) qui est chargée d'acheminer le sang du patient vers le filtre (2), une branche veineuse (4) qui est chargée d'acheminer le sang du filtre (2) vers le patient, une pluralité de pompes (9, 10) adaptées pour faire circuler à la fois le sang et la solution de dialyse, un dispositif de mesure de la saturation en oxygène (13) appliqué à ladite branche artérielle (3) et une unité de commande/contrôle (12) adaptée pour recevoir des informations dudit dispositif de mesure de la saturation en oxygène (13) ; ladite machine étant **caractérisée en ce qu'**elle comprend un dispositif d'ultra-filtrage isolé (14) disposé le long de la branche artérielle (3) et un dispositif de mesure de concentration sodique du sang du patient (16) qui est appliqué au plasma ultra-filtré produit par ledit dispositif d'ultra-filtrage isolé (14) et adapté pour donner des informations à ladite unité de commande/contrôle (12) ; ladite unité de commande/contrôle (12) étant connectée à un dispositif de préparation de solution de dialyse (7) adapté pour modifier la composition et la température de la solution de dialyse elle-même, et à une pompe (10) appliquée à ladite ramification de sortie (6) de la solution de dialyse adaptée pour régler l'écoulement de l'ultra-filtré prélevé chez le patient ; ladite unité de commande/contrôle (12) ayant des algorithmes d'adaptation de processus d'hémodiafiltration mis en oeuvre en fonction de la saturation en oxygène du sang du patient et intervenant sur un composant d'ultra-filtrage dudit filtre (2), sur la concentration sodique et sur la température de la solution de dialyse ; ladite unité de commande/contrôle (12) ayant en outre des algorithmes mis en oeuvre pour adapter la conductivité et la concentration sodique dans la solution de dialyse en fonction du niveau de sodium dans le sang du patient et intervenant sur la solution de dialyse par le biais du dispositif de préparation de solution de dialyse (7) et sur ladite pompe (10) appliquée à ladite ramification de sortie pour rétablir la valeur sodique correcte dans le sang du patient.

2. Machine selon la revendication 1, **caractérisée en ce que** ledit dispositif de mesure de saturation en oxygène du sang (13) comprend des moyens optiques destinés à détecter l'oxyhémoglobine dans l'hémoglobine totale.

3. Machine selon la revendication 1 ou 2,
**caractérisée en ce qu'**elle comprend un dispositif (18) pour mesurer la pression artérielle du patient ; ledit dispositif (18) pour mesurer la pression artérielle du patient étant connecté à ladite unité de commande/contrôle (12).

4. Machine selon l'une quelconque des revendications précédentes, **caractérisée en ce qu'**elle comprend un dispositif de mesure du rythme cardiaque du patient (17) ; ledit dispositif de mesure de fréquence cardiaque du patient (17) étant connecté à ladite unité de commande/contrôle (12).
